# EUROPÄISCHE PATENTSCHRIFT

(11) **EP 2 427 247 B1**
(45) Veröffentlichungstag und Bekanntmachung des Hinweises auf die Patenterteilung: **18.12.2013**
(21) Anmeldenummer: 10710575.1
(22) Anmeldetag: 22.03.2010
(51) Int. Cl.: A61Q 5/08, A45D 19/00, A61K 8/02, A61K 8/22, A61K 8/42, A61K 8/49, A61K 8/81

(54) **FOLIENAPPLIKATOR**
FOIL APPLICATOR
APPLICATEUR EN FEUILLE

(30) Priorität: 07.05.2009 DE 102009002908
(43) Veröffentlichungstag der Anmeldung: 14.03.2012
(73) Patentinhaber: Henkel AG & Co. KGaA, 40589 Düsseldorf (DE)
(72) Erfinder: KLEEN, Astrid, 20457 Hamburg (DE); RATH, Susanne, 20359 Hamburg (DE); WELZ, Carolin, 22459 Hamburg (DE)
(86) Internationale Anmeldenummer: PCT/EP2010/053660
(87) Internationale Veröffentlichungsnummer: WO 2010/127902

(56) Entgegenhaltungen:
- EP-A2- 1 792 641
- EP-A2- 1 958 532
- WO-A1-93/10687
- DE-A1- 19 543 989
- DE-A1-102006 008 542
- US-A- 5 931 168
- US-A1- 2003 135 937

## Beschreibung

Die vorliegende Anmeldung betrifft einen folienartigen Applikator zur insbesondere selektiven Farbaufhellung keratinischer Fasern, insbesondere menschlicher Haare sowie die Verwendung solcher Applikatoren zur Erzeugung gezielter Aufhelleffekte im Haar.

Im Laufe der Zeit und insbesondere unter Einwirkung von äußeren Einflüssen wie Licht oder atmosphärischen Schadstoffen verliert oder verändert das Haar seine natürliche Farbe und seinen Glanz bzw. Schimmer. Insbesondere die Aufhellung von Haaren steht dabei im besonderen Focus des Anwenders. Aus diesem Grund finden Aufhellmittel breite Anwendung, die entweder im Friseurbereich oder durch die Heimanwendung genutzt werden. Bei der Haarfärbung - insbesondere bei der Haarfärbung durch die Heimanwendung - treten mehrere Probleme auf. Einerseits werden natürliche Farbnuancierungen vollständig überdeckt, so dass multitonale Färbungen schwer zu realisieren sind. Andererseits ist die selektive Aufhellung einzelner Haarpartien, wie beispielsweise Faserbündeln, Strähnen und Scheitel, ohne geeignete Hilfsmittel nur schwierig zu erreichen. Häufig erfordert die Anwendung geeigneter Hilfsmittel wie Applikatoren oder Bürsten jedoch viel Erfahrung und Geschick.

Um dem Haar ein natürlicheres Aussehen zu verleihen, wird vorgeschlagen, ungefärbte oder gefärbte Haare durch gezielte Anwendung von Oxidationsmitteln partiell zu entfärben. Die Haarpartien ("Strähnchen"), auf die die Oxidationsmittel aufgetragen werden, bleichen dabei mindestens anteilsweise aus, woraus eine multitonale Haarfarbe resultiert. Die Applikation des Oxidationsmittels erfolgt dabei mit einer Bürste oder einem Pinsel, wobei die zu behandelnden Haare gegebenenfalls mit Aluminiumfolie oder einer so genannten "Strähnchenhaube" selektiert werden.

Im Stand der Technik, insbesondere WO1993/010687A1 wurde daher vorgeschlagen, so genannte Strähnchenfolien oder Strähnchenpapiere zur Erstellung von Strähnchen einzusetzen. Dabei handelt es sich um flexible Substrate, die mit einem Aktivator oder Aufhellmittel imprägniert sind. Die imprägnierten Substrate werden vor der Anwendung mit einem Oxidationsmittel behandelt, um die eigentliche Aufhellzubereitung herzustellen, und zur Anwendung um die aufzuhellende Strähne gewickelt.

Die Anmischung auf der Folie birgt für den Anwender einige Nachteile. Zum einen muss sicher-gestellt werden, dass alle Folien mit der gleichen Menge an Oxidationsmittel behandelt werden. Andererseits ist nach der Anmischung der Oxidationsmittelzubereitung die Zeit, in der die Anwendung auf der Strähne erfolgen kann, eingeschränkt. Unterschiedliche Zubereitungszusammensetzungen und/oder variierende Anwendungszeiträume können jedoch zu uner-wünschten und uneinheitlichen Aufhellergebnissen. Daher besteht weiterhin der Bedarf nach für insbesondere ungeübte Anwender einfach anzuwendenden Applikatorformen zur selektiven Aufhellung von Haarpartien, insbesondere zur Erzeugung sogenannter Highlights oder Strähnchen.

Es wurde nun gefunden, dass zur gezielten und selektiven Anwendung von aufhellenden Mitteln ein spezieller Applikator in Folienform vorteilhaft eingesetzt werden kann, wenn es sich bei dem Applikator um eine mit einem aufhellenden Mittel beschichteten Folie handelt, die um das nasse Haar gewickelt wird und durch die Feuchtigkeit des Haares das Aufhellmittel auf dem Haar wirksam wird.

Diese Form von Folien-Appilkator unterscheidet sich vom Stand der Technik dahingehend vorteilhaft, dass ein aufwendiges, zeitintensives Vormischen des Mittels auf der Folie entfällt. Gegebenenfalls auftretende Probleme mit Mengenverhältnissen zwischen Aktivator und Oxidationsmittel oder Homogenisierung des Mittels werden ebenfalls vermieden. Außerdem ist mit den erfindungsgemäßen Applikatoren die Anwendungszeit für das Blondiermittel präzise einzuhalten, da der Aufhellvorgang erst mit Kontakt des Folien-Applikators mit dem Haar oder der Haarsträhne einsetzt Somit kann der Anwender die Einwirkzeit des Aufhellmittels genau bestimmen.
Schließlich kann der Anwender über die Wahl einer Strähne den Ort der Aufhellung im Haar sehr präzise festlegen und das Aufhellmittel spezifisch am gewünschten Wirkort applizieren, was bei einer Auftragung mit Pinsel oder Bürste nur unzureichend möglich ist

Ein erster Gegenstand der vorliegenden Erfindung ist daher ein Folien-Applikator zur selektiven Aufhellung keratinischer Fasern, umfassend ein blattförmiges Substrat sowie eine darauf aufgebrachte kosmetische Blondierzubereitung, die dadurch gekennzeichnet ist, dass die Blondierzubereitung wasserfrei ist und mindestens ein festes Peroxodisulfat-Salz sowie mindestens ein festes Anlagerungsprodukt von Wasserstoffperoxid an organische oder anorganische Verbindungen sowie weiterhin mindestens ein Polymer, ausgewählt aus Cellulose, Celluloseethern, Alginsäure, Xanthan-Gum, Gummi arabicum, Polyvinylalkohol oder Umsetzungs-Produkten von Guar-Gum mit Chloressigsäure, mit Ethylenoxid oder mit Propylenoxid, enthält. Die Begriffe "Folien-Applikator", "Strähnchenfolie" bzw. "Applikator" werden nachstehend synonym verwendet.
Der erfindungsgemäße Applikator umfasst ein flexibles Substrat. Das flexible Substrat kann dabei jedwede Form annehmen und aus jedwedem Material oder aus jeder Materialmischung bestehen, das bzw. die flexibel ist. "Flexibel" im Sinne der vorliegenden Erfindung sind Substrate, die hinsichtlich Form und Materialien so ausgebildet sind, dass sie mit manueller Kraft verformt werden können, wobei eine reversible Verformung bevorzugt ist Die erfindungsgemäßen flexiblen Substrate sind blattförmig. "Blattförmig" im Sinne der vorliegenden Erfindung bedeutet, dass die räumliche Ausdehnung des Substrates entlang einer Raumrichtung deutlich kleiner ist als entlang der beiden anderen Raumrichtungen, wobei die geringste räumliche Ausdehnung des Substrats im Rahmen der vorliegenden Erfindung als "Höhe" bezeichnet wird, während die entsprechende Ausdehnung des Substrats in die hierzu orthogonal stehenden Raumrichtungen als "Breite" und "Länge" bezeichnet werden. Ist die räumliche Ausdehnung des Substrats entlang der zur Höhe orthogonal stehenden Raumrichtungen unterschiedlich, so wird im Rahmen der vorliegenden Erfindung die kleinere räumliche Ausdehnung als "Breite" bezeichnet, die größere als "Länge".

Bei bevorzugten erfindungsgemäßen Substraten beträgt das Verhältnis von Breite zu Höhe 10 zu 1 bis 100.000 zu 1, vorzugsweise 50 zu 1 bis 50.000 zu 1 und insbesondere 100 zu 1 bis 1000 zu 1. Besonders bevorzugte erfindungsgemäße blattförmige Substrate sind dadurch gekennzeichnet, dass ihre Höhe 10 bis 1000 µm, vorzugsweise 25 bis 500 µm und insbesondere 40 bis 250 µm beträgt. Länge und Breite bevorzugter blattförmiger Substrate liegen vorzugsweise im Bereich zwischen 1 und 30 cm, besonders bevorzugt zwischen 2 und 20 cm, weiter bevorzugt zwischen 3 und 15 cm und insbesondere zwischen 4 und 12 cm.

Eine bevorzugte Ausführungsform des erfindungsgemäßen Folien-Applikators ist dadurch gekennzeichnet, dass das blattförmige Substrat eine Dicke von 5 µm bis 10 mm, vorzugsweise von 10 µm bis 1 mm, besonders bevorzugt von 15 µm bis 0,5 mm und insbesondere von 20 µm bis 0,1 mm, aufweist.

Im Hinblick auf eine stabile Formgebung beim Aufhellprozess und unter Berücksichtigung der Tatsache, dass sich das blattförmige Substrat ohne weitere mechanische Hilfsmittel stabil um eine Haarsträhne legen lassen soll, ist der Einsatz von Aluminiumfolien besonders bevorzugt. Da Aluminiumfolie chemisch gegenüber der anwendungsbereiten Blondierzusammensetzung nicht in hohem Maße stabil ist, empfehlen sich mehrlagige blattförmige Substrate, beispielsweise doppellagige Folien aus Kunststoff und Aluminium. Hierzu eignen sich insbesondere Laminate, bei denen die einzelnen Schichten miteinander verklebt sind.

Erfindungsgemäß bevorzugte Folien-Applikatoren sind dadurch gekennzeichnet, dass das blattförmige Substrat wenigstens eine Schicht aus Aluminiumfolie umfasst.

Erfindungsgemäße blattförmige Substrate können aus unterschiedlichen Materialien oder Materialgemischen bestehen, wobei sich Papier, Karton und Folien bewährt haben.

Papier ist ein flächiger, im Wesentlichen aus Fasern meist pflanzlicher Herkunft bestehender Werkstoff, der durch Entwässerung einer Faserstoffaufschwemmung auf einem Sieb gebildet wird. Dabei entsteht ein Faserfilz, der anschließend verdichtet und getrocknet wird. Die flächenbezogene Masse von Papier liegt üblicherweise ≤ 225 g/m². Karton ist ein flächiger, im Wesentlichen aus Fasern meist pflanzlicher Herkunft bestehender Werkstoff, der hinsichtlich der flächenbezogenen Masse (150-600 g/m²) sowohl in das Gebiet der Papiere als auch in das der Pappen hineinreicht. Er ist steifer als Papier und wird im Allgemeinen aus hochwertigeren Stoffen als Pappe hergestellt. Karton wird als endlose Bahn gefertigt. Die Be-nennung Karton ist nur im deutschen Sprachgebrauch üblich. Folien (von lateinisch: folium = Blatt) sind dünne, flächige, flexible, aufwickelbare Bahnen aus Metallen (z.B. Blattgold, Aluminium, Zinn-Folie = Stanniol) oder Kunststoffen (z.B. Celluloseacetat, PVC, Polyethylen etc.). Besonders dünne Folien nennt man auch Filme. Metallfolien erhält man durch Walzen oder Schlagen, Kunststoff-Folien durch Giessen, Kalandrieren oder Extrudieren, das als Blasformen ausgeführt wird, wobei Schlauch- bzw. BlasFolien entstehen, die aufgeschnitten werden.

Erfindungsgemäß besonders geeignete Folienmaterialien sind Kunststoffe, wobei sich thermoplastische Kunststoffe besonders bewährt haben. Unter "Kunststoffen" versteht man allgemeinen Materialien, deren wesentliche Bestandteile aus solchen makromolekularen organischen Verbindungen bestehen, die synthetisch oder durch Abwandeln von Naturprodukten entstehen. Sie sind in vielen Fällen unter bestimmten Bedingungen (Wärme und Druck) schmelz- und formbar. Kunststoffe sind generell nach allen für die Synthese von Polymeren üblichen Polyreaktionen (Polyadditionen, Polykondensationen und Polymerisationen) und Polymer-analogen Reaktionen herstellbar. Erfindungsgemäß besonders bevorzugte blattförmige Substrate werden aus einem oder mehreren der folgenden Kunststoffe hergestellt:
Cellulosenitrat, Celluloseacetat, Cellulosemischester, Celluloseether, Polyamid, Polycarbonat, Polyester, Polyvinylacetal, Polyethylen, Polypropylen, Poly-1-buten, Polybutadien, Polyisopren, Poly-4-methyl-1-penten, lonomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, lineare Polyurethane und chlorierte Polyether.

Die wasserfreie Blondierzubereitung, die auf das blattförmige Substrat aufgebracht ist, kann in fester, halbfester, flüssiger, disperser, emulgierter, suspendierter, oder gelförmiger Form vorliegen. In einer Ausführungsform liegt die Blondierzubereitung in fester Form vor.

Wasserfrei im Sinne der Erfindung bedeutet, dass die Blondierzubereitung einen Wassergehalt von kleiner als 5,0 Gew.-%, bevorzugt kleiner als 1,0 Gew.-%, insbesondere kleiner als 0,1 Gew.-% und ganz besonders bevorzugt kleiner als 0,05 Gew.-%, jeweils bezogen auf das Gesamtgewicht der Blondierzubereitung, besitzt.

Dabei kann eine vollflächige Beschichtung des blattförmigen Substrates vorliegen, oder es können nur bestimmte Areale des Substrates beschichtet sein. Auch die Menge und die Schichtdicke der Zubereitung können je nach gewünschter Applikationsart variieren. Vorzugsweise werden die Massen von Substrat und Zubereitung aufeinander abgestimmt. Hier sind erfindungsgemäße Strähnchenfolien bevorzugt, bei denen die Blondierzubereitung mindestens 10 Gew.%, vorzugsweise mindestens 25 Gew.% und insbesondere mindestens 50 Gew.-% des Gesamtgewichtes der Strähnchenfolie ausmacht.

Die Blondierzubereitung zur Beschichtung der Folie enthält mindestens ein festes Anlagerungsprodukt von Wasserstoffperoxid an organische oder anorganische Verbindungen und mindestens ein festes Peroxodisulfatsalz.

Wasserstoffperoxid kann mit vielen anorganischen und organischen Verbindungen Anlagerungsprodukte bilden. Hierzu zählen Anlagerungsprodukten an Harnstoff (Harnstoff-Wasserstoffperoxid-Additionsverbindung, UHP, Hydroperit, Perhydrol-Harnstoff, Wasserstoffperoxid-Carbamid, Percarbamid), Melamin (Melaminperoxid) und Polyvinylpyrrolidinon (PVP·n H₂O₂) sowie Percarbonate, insbesondere Natriumpercarbonat und Magnesiumpercarbonat, und Perborate, insbesondere Natriumperborat.

In einer bevorzugten Ausführungsform enthält die Blondierzubereitung ein festes Anlagerungsprodukt von Wasserstoffperoxid an organische oder anorganische Verbindungen, welches ausgewählt ist aus Anlagerungsprodukten an Harnstoff, Melamin und Polyvinylpyrrolidinon.

Die Blondierzubereitungen enthalten das feste Anlagerungsprodukt von Wasserstoffperoxid an organische oder anorganische Verbindungen in einer Menge von 0,1 bis 60 Gew.-%, bevorzugt von 1 bis 50 Gew.% und besonders bevorzugt von 2 bis 30 Gew.%, jeweils berechnet als 100%iges H₂O₂ und bezogen auf das Gesamtgewicht der Blondierzubereitung.

Als weiteren wesentlichen Inhaltsstoff enthalten die Blondierzubereitungen mindestens ein festes Peroxodisulfat-Salz zur Aktivierung und Verstärkung der Aufhellwirkung der Zubereitung.

Eine Ausführungsform der vorliegenden Erfindung ist dadurch gekennzeichnet, dass das feste Peroxodisulfat-Salz ausgewählt ist aus der Gruppe, die gebildet wird aus Ammoniumperoxodisulfat, Kaliumperoxodisulfat und Natriumperoxodisulfat.

Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfat-Salze sind dabei Kombinationen aus Ammoniumperoxodisulfat und Kaliumperoxodisulfat und/oder Natriumperoxodisulfat.

Die Blondierzubereitungen enthalten feste Peroxodisulfat-Salze in einer Menge von 0,1 bis 40 Gew.%, bevorzugt von 1 bis 30 Gew.% und besonders bevorzugt von 2 bis 20 Gew.%, jeweils bezogen auf das Gesamtgewicht der Blondierzubereitung.

Die efindungsgemäßen Blondiermittel können zusätzlich zu den festen Peroxodisulfatsalzen einen weiteren Bleichkraftverstärker enthalten.

Geeignete Bleichkraftverstärker sind anorganische Peroxoverbindungen, ausgewählt aus Ammoniumperoxomonosulfat, Alkalimetallperoxomonosulfaten, Alkalimetallperoxodiphosphaten und Erdalkalimetallperoxiden. Besonders bevorzugte Bleichkraftverstärker sind Kaliumhydrogen-peroxomonosulfat, Kaliumperoxodiphosphat, Magnesiumperoxid und Bariumperoxid.

Als Bleichverstärker können ebenfalls Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Natrium- und Kalium-) sowie Erdalkalimetall- (insbesondere Magnesium- und Calcium-), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichverstärker verwendet werden.

Weiterhin sind Alkylcarbonate, -carbamate, Silylcarbonate sowie -carbamate erfindungsgemäße geeignete Bleichverstärker.

Weiterhin erfindungsgemäß einsetzbare Bleichkraftverstärker können aus stickstoffhaltigen, gegebenenfalls kationischen Heterocyclen. Als Beispiel eines stickstoffhaltigen heterocyclischen Bleichkraftverstärkers ist insbesondere Imidazol zu nennen.

Bevorzugt werden stickstoffhaltige, heterocyclische Bleichkraftverstärker aber unter kationischen Heterocyclen gewählt. Hierbei sind insbesondere kationische Pyridine, Pyrimidine, Pyrazine, Pyridazine, Triazine, Imidazole, Thiazole, Oxazole, Isoxazole, Chinoline, Isochinoline sowie deren partielle oder vollständig gesättigten Analoga.

Besonders bevorzugte stickstoffhaltige, heterocyclische Bleichkraftverstärker sind die quaternierten Kationen von Pyridinen und 3,4-Dihydroisochinolinen.

Erfindungsgemäß bevorzugte Bleichkraftverstärker vom Typ der Pyridinium-Salze sind dabei 4-Acetyl-1-methylpyridinium-p-toluolsulfonat, 4-Acetyl-1-methylpyridiniumbenzolsulfonat, 4-Acetyl-1-methylpyridiniumbromid, 4-Acetyl-1-methylpyridiniumhydrogensulfat, 4-Acetyl-1-allylpyridinium-p-toluolsulfonat, 4-Acetyl-1-allylpyridiniumbenzolsulfonat, 4-Acetyl-1-allylpyridiniumbromid, 4-Acetyl-1-allylpyridiniumhydrogensulfat, 4-Acetyl-1-(2-hydroxyethyl)pyridinium-p-toluolsulfonat, 4-Acetyl-1-(2-hydroxyethyl)pyridiniumbenzolsulfonat, 4-Acetyl-1-(2-hydroxyethyl)pyridiniumbromid, 4-Acetyl-1-(2-hydroxyethyl)pyridiniumhydrogensulfat, 4-Acetyl-1-(2-oxopropyl)pyridinium-p-toluolsulfonat, 4-Acetyl-1-(2-oxopropyl)pyridiniumbenzolsulfonat, 4-Acetyl-1-(2-oxopropyl)pyridiniumbromid, 4-Acetyl-1-(2-oxopropyl)pyridiniumhydrogensulfat, 4-Acetyl-1-ethylpyridinium-p-toluolsulfonat, 4-Acetyl-1-ethylpyridiniumbenzolsulfonat, 4-Acetyl-1-ethylpyridiniumbromid, 4-Acetyl-1-ethyl-pyridiniumhydrogensulfat, 4-Acetyl-1-(2-methyl-prop-2-enyl)pyridinium-p-toluolsulfonat, 4-Acetyl-1-(2-methyl-prop-2-enyl)pyridiniumbenzolsulfonat, 4-Acetyl-1-(2-methyl-prop-2-enyl)pyridinium-bromid, 4-Acetyl-1-(2-methyl-prop-2-enyl)pyridiniumhydrogensulfat, 4-Acetyl-1-benzylpyridinium-p-toluolsulfonat, 4-Acetyl-1-benzylpyridiniumbenzolsulfonat, 4-Acetyl-1-benzylpyridiniumbromid, 4-Acetyl-1-benzylpyridiniumhydrogensulfat, 4-Acetyl-1-(2-methoxyethyl)pyridinium-p-toluolsulfonat, 4-Acetyl-1-(2-methoxyethyl)pyridiniumbenzolsulfonat, 4-Acetyl-1-(2-methoxyethyl)pyridiniumbromid oder 4-Acetyl-1-(2-methoxyethyl)pyridiniumhydrogensulfat sowie 2-Acetyl-1-methylpyridinium-p-toluolsulfonat, 2-Acetyl-1-methylpyridiniumbenzolsulfonat, 2-Acetyl-1-methylpyridiniumbromid, 2-Acetyl-1-methylpyridiniumhydrogensulfat, 2-Acetyl-1-allylpyridinium-p-toluolsulfonat, 2-Acetyl-1-(2-hydroxyethyl)pyridinium-p-toluolsulfonat, 2-Acetyl-1-(2-oxopropyl)pyridinium-p-toluolsulfonat und 2-Acetyl-1-(2-methoxyethyl)pyridinium-p-toluolsulfonat.

Erfindungsgemäß weiterhin bevorzugte Bleichkraftverstärker vom Typ der 3,4-Dihydroisochinoline sind dabei N-Methyl-3,4-dihydroisochinolinium-p-toluolsulfonat, N-Methyl-3,4-dihydroiso-chinoliniumbenzolsulfonat, N-Methyl-3,4-dihydroisochinoliniumhydrogensulfat, N-Allyl-3,4-dihydro-isochinolinium-p-toluolsulfonat, N-Allyl-3,4-dihydroisochinoliniumbenzolsulfonat, N-Allyl-3,4-dihydroisochinoliniumbromid, N-Allyl-3,4-dihydroisochinoliniumacetat, 3,4-Dihydro-2-(3-hydroxy-propyl)isochinolinium-p-toluolsulfonat, 3,4-Dihydro-2-(3-hydroxypropyl)isochinoliniumbenzol-sulfonat, 3,4-Dihydro-2-(3-hydroxypropyl)isochinoliniumbromid, 3,4-Dihydro-2-(3-hydroxypropyl)iso-chinoliniumacetat, 3,4-Dihydro-2-(2-hydroxyethyl)isochinolinium-p-toluolsulfonat, 3,4-Dihydro-2-(2-hydroxyethyl)isochinoliniumbenzolsulfonat, 3,4-Dihydro-2-(2-hydroxyethyl)isochinoliniumbromid oder 3,4-Dihydro-2-(2-hydroxyethyl)isochinoliniumacetat.

Die neben oder anstelle von Peroxoverbindungen eingesetzten Bleichkraftverstärker sind in den erfindungsgemäßen, kosmetischen Mitteln bevorzugt in Mengen von 0,5 bis 30 Gew.%, insbesondere in Mengen von 2 bis 20 Gew.%, jeweils bezogen auf das Gesamtgewicht des anwendungsbereiten Mittels, enthalten.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.% bis 15 Gew.%, besonders bevorzugt in Mengen von 0,15 Gew.% bis 10 Gew.-% und ganz besonders bevorzugt in Mengen von 0,2 Gew.% bis 5 Gew.%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt.
Erfingdungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt.

Insbesondere Metasilicate, die sich gemäß vorstehender Formel durch das Verhältnis zwischen n und der Summe aus m und p von 5 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ, ist dabei besonders bevorzugt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfingdungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW und Portif^{®} W und von der Firma Akzo unter der Bezeichnung Britesil^{®} C20 vertrieben.
Die Blondierzubereitung enthält zusätzlich zur Verbesserung der Beaufschlagung auf die Folie weiterhin mindestens ein Polymer, ausgewählt aus Cellulose, Celluloseethern, Alginsäure, Xanthan-Gum, Gummi arabicum, Polyminylalkohol oder Umsetzungsprodukten von Guar-Gum mit Chloressigsäure, mit Ethylenoxid oder mit Propylenoxid.
Es hat sichherausgestellt, dass wasserlösliche Polymere besonders vorteilhaft einsetzbar sind. Solche Blondierzubereitungen werden von der Nässe der umwickelten Haarsträhnen besonders gut von der Folie aufgelöst und können sich so besonders gut zwischen den Haarfasem verteilen. Dies führt zu einem gleichmäßigen Aufhellergebnis. Erfindungsgemäße Polymere sind daher Cellulose, Celluloseether, wie Methylcellulose, Hydroxypropylcellulose, Carboxymethylcellulose, Polyelektrolyte, wie Alginsäure, Xanthan-Gum, Gummi arabicum, oder modifizierte, natürliche Verdickungsmittel, wie Umsetzungsprodukte von Guar-Gum mit Chloressigsäure, mit Ethylenoxid oder mit Propylenoxid, oder Polyvinylalkohol.

Bei der Entfärbung von Haaren, insbesondere mit dunkler Ausgangsfarbe, kann es zu uner-wünschten Farbverschiebungen kommen. Dies liegt darin begründet, dass sie natürliche Haarfarbe durch Melanine in der Cortex der Haarfaser bestimmt wird, wobei das Verhältnis von der zwei Pigmentklassen, Eumelanine mit bräunlich-schwarzen Tönen und Pheomelanine mit rötlich-orangen Tönen, die eigentliche Haarfarbe bestimmt. Beim Blondierprozess werden die natürlichen Melanin-Farbstoffe durch oxidative Einwirkung zerstört und so eine Entfärbung der Fasern erreicht Aufgrund der unterschiedlichen oxidativen Zerstörungsrate der verschiedenen Melaninpigmentklassen werden Haare jedoch nicht gleichmäßig entfärbt. In dunkleren Fasern mit hohem Melaningehalt verbleibt zumeist ein bestimmter Anteil an Farbstoffen, der sich häufig in gelblichen bis rötlichen Nuancen niederschlägt Daher kommt es insbesondere beim Blondieren von dunkleren Haaren zu einer Farbverschiebung in Richtung warmer Töne.

Üblicherweise sind solche Farbverschiebungen in Richtung warmer Töne bei dem Anwender unerwünscht. Daher wird dieser Farbverschiebung zumeist durch eine Tönung mit der ent-sprechenden Komplementärfarbe gemäß der Farbenlehre entgegengewirkt. Ziel ist dabei ein silbrig-kühler Eindruck des Bleichergebnisses. Der Fachmann spricht in diesem Zusammenhang von einer Mattierung. Je nach Ausgangshaarfarbe muss dabei eine angepasste Tönungsmittelmischung eingesetzt werden, um entsprechend rötlichere Farbverschiebungen durch grünlichere Tönungsmittel oder gelblichere Farbverschiebungen durch eher violette Tönungsmittel auszugleichen.

Als Tönungsmittel eignen sich insbesondere direktziehende Farbstoffe, insbesondere solche, die unter den oxidativen und stark alkalischen Bedingungen einer Blondierung hinreichend stabil sind.

In einer weiteren Ausführungsform enthält die Blondierzubereitung daher zusätzlich eine Kombination aus mindestens einem blauen direktziehenden Farbstoff und einem roten direktziehenden Farbstoff, wobei das Gewichtsverhältnis zwischen der Summe aller blauen direktziehenden Farbstoffe und der Summe aller roten direktziehenden Farbstoffe einen Wert von größer oder gleich 1 besitzt. Dadurch ist es möglich, unerwünschte Farbverschiebungen in Richtung rosa-/roséfarbene Nuancen zu vermieden.

Erfindungsgemäß bevorzugte Mittel sind dadurch gekennzeichnet, dass das Gesamtgewicht aller blauen direktziehenden Farbstoffe größer als das Gesamtgewicht aller roten direktziehenden Farbstoffe ist. Bevorzugte Mittel sind daher dadurch gekennzeichnet, das Gewichtsverhältnis zwischen der Summe aller blauen direktziehenden Farbstoffe und der Summe aller roten direktziehenden Farbstoffe einen Wert von 1 bis 100, bevorzugt von 1,5 bis 10 und besonders bevorzugt von 2 bis 4 besitzt.

Prinzipiell sind der Wahl der direktziehenden Farbstoffe keine Grenzen gesetzt. Direktziehende Farbstoffe werden üblicherweise in anionische, kationische und nichtionische direktziehende Farbstoffe unterteilt. Erfindungsgemäß einsetzbare direktziehende Farbstoffe sind dabei Nitrophenylendiamine, Nitroaminophenole, Azofarbstoffe, Anthrachinone oder Indophenole, wenn diese direktziehenden Farbstoffe eine hinreichende Stabilität gegenüber den harschen Bedingungen des Blondierprozesses besitzen.

Bevorzugt enthält das Mittel als blauen direktziehenden Farbstoff mindestens einen anionischen direktziehenden Farbstoff, insbesondere ausgewählt aus Verbindungen mit der Bezeichnung Bromphenolblau oder Tetrabromphenolblau.

Bevorzugt enthält das Mittel als roten direktziehenden Farbstoff mindestens einen anionischen direktziehenden Farbstoff, bevorzugt aus der Gruppe der Fluorescin-Farbstoffe. Besonders bevorzugte, rote direktziehende Farbstoffe sind unter den Bezeichnungen Acid Red 92, Acid Red 98, Acid Red 94, Acid Red 87 und Acid Red 51 bekannt. Ganz besonders bevorzugt ist dabei Acid Red 92 (auch D&C RED No. 28 oder Phloxin B).

Erfindungsgemäß bevorzugte Farbstoffkombinationen zur Mattierung in der Blondierzubereitung sind solche, die mindestens die Kombination aus
- Tetrabromphenolblau und Acid Red 92,
- Tetrabromphenolblau und Acid Red 98,
- Tetrabromphenolblau und Acid Red 94,
- Tetrabromphenolblau und Acid Red 87 oder
- Tetrabromphenolblau und Acid Red 51 enthalten.

Es kann erfindungsgemäß weiterhin bevorzugt sein, wenn die Blondierzubereitung weitere direktziehende Farbstoffe enthält. Besonders bevorzugt enthält das Mittel als weiteren direktziehenden Farbstoff einen gelben und/oder orangen Farbstoff. Dies ist insbesondere dann vorteilhaft, wenn unerwünschte rötliche Farbverschiebungen beim Blondierprozess auftreten.

Bevorzugt werden die gelben Farbstoffe ausgewählt aus geeigneten gelben Nitrofarbstoffe, wie1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxy-ethyl)amino]-5-nitrobenzol (HC Yellow 5), 4-[(2,3-Dihydroxypropyl)[amino]-3-nitro-1-trifluormethyl-benzol (HC Yellow 6), 2-[Di(2-hydroxyethyl)amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitro-benzol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)-amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluor-methyl-benzol (HC Yellow 13), 4-[(2-Hydroxyethyl)-amino]-3-nitro-benzonitril (HC Yellow 14), 4-[(2-Hydroxyethyl)amino]-3-nitro-benzamid (HC Yellow 15) 3-[(2-Hydroxyethyl)amino]-4-methyl-1-nitrobenzol, 4-Chlor-3-[(2-hydroxyethyl)amino]-1-nitrobenzol. Geeignete gelbe oder orange Chinonfarbstoffe sind insbesondere 2-[(2-Aminoethyl)amino]-9,10-anthrachinon (HC Orange 5) und 2-Hydroxy-1,4-naphthochinon (C.I. 75,480, Natural Orange 6). Auch neutrale, gelbe oder orange Azofarbstoffe können erfindungsgemäß vorteilhaft eingesetzt werden, insbesondere 4-[(4-Amino-phenyl)azo]-1-[di(2-hydroxyethyl)amino]-3-methylbenzol (HC Yellow 7), 2,6-Diamino-3-[(pyridin-3-yl)azo]pyridin, 2-{(4-(Acetylamino)phenyl]azo}-4-methylphenol (C.I. 11855; Disperse Yellow 3), 4-[(4-Nitrophenyl)azo]-anilin (C.I. 11,005; Disperse Orange 3). Als gelbe oder orange, anionische direktziehende Farbstoffe eignen sich insbesondere 6-Hydroxy-5-[(4-sulfophenyl)azo]-2-naphthalin-sulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)-benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobenzolsulfonsäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4-Dihydroxyphenyl)azo]benzolsulfonsäure-natriumsalz (C.I. 14,270; Acid Orange 6), 4-[(2-Hydroxynaphth-1-yl)azo]benzolsulfonsäure-natriumsalz (C.I. 15,510; Acid Orange 7), 4-[(2,4-Dihydroxy-3-[(2,4-dimethylphenyl)azo]-phenyl)azo]benzolsulfonsäure-natriumsalz (C.I. 20,170; Acid Orange 24). Als kationische direktziehende Farbstoffe eignen sich insbesondere 3-[(4-Amino-2,5-dimethoxyphenyl)azo]-N,N,N-trimethylbenzolaminiumchorid (C.I. 12,605, Basic Orange 69), Di[4-(dimethylamino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]pyrazol-5-on-chlorid (C.I. 12,719; Basic Yellow 57). Bevorzugte gelbe oder orange, kationische direktziehende Farbstoffe sind Basic Yellow 87 und Basic Orange 31. Besonders bevorzugte gelbe oder orange, nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Yellow 13, HC Orange 1 und Disperse Orange 3, insbesondere HC Yellow 13.

Es ist dabei bevorzugt, wenn die direktziehenden Farbstoffe zu 0,0001 Gew.% bis 5,0 Gew.-%, insbesondere zu 0,001 Gew.-% bis 2,5 Gew.-% und ganz besonders bevorzugt zu 0,01 Gew.-% bis 1,5Gew.-%, jeweils bezogen auf das Gesamtgewicht des anwendungsbereiten Mittels, in der Blondierzubereitung vorliegt.

Weiterhin hat es sich als vorteilhaft erweisen, wenn die Blondiermittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, die über mehrere Koordinationsstellen mit einem Metallion Bindungen eingehen.

Im Rahmen der vorliegenden Erfindung können alle Komplexbildner des Standes der Technik eingesetzt werden. Diese können unterschiedlichen chemischen Gruppen angehören. Vorzugsweise werden einzeln oder im Gemisch miteinander eingesetzt:
a) Polycarbonsäuren, bei denen die Summe der Carboxyl- und gegebenenfalls Hydroxylgruppen mindestens 5 beträgt wie Gluconsäure;
b) stickstoffhaltige Mono- oder Polycarbonsäuren wie Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibemsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfo-bernsteinsäure) (DDS), Ethylendicysteinsäure (EDC), Ethylendiamin-N-N'-bis(ortho-hydroxyphenyl)essigsäure (EDDHA), N-2-Hydroxyethylamin-N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Salze und/oder Derivate;
c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate;
d) Aminophosphonsäuren wie Ethylendiamintetra(methylenphosphonsäure) (EDTMP), Di-ethylen-triaminpenta(methylenphosphonsäure) (DTPMP) sowie deren höhere Homologe, oder Nitrilotri(methylenphosphonsäure);
e) Phosphonopolycarbonsäuren wie 2-Phosphonobutan-1,2,4-tricarbonsäure; sowie
f) Cyclodextrine und
g) Alkalistannate (Natriumstannat), Alkalipyrophosphate (Tetranatriumpyrophosphat, Dinatriumpyrophosphat), Alkaliphosphate (Natriumphosphat), und Phosphorsäure.

Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Erfindungsgemäß bevorzugte Blondiermittel für keratinische Fasern sind dadurch gekennzeichnet, dass sie einen alkalischen pH-Wert besitzen. Eine weitere bevorzugte Ausführungsform der vorliegenden Erfindung besteht darin, dass das anwendungsbereite Mittel einen pH-Wert zwischen 7,0 und 12,0, bevorzugt zwischen 8,0 und 11,0 besitzt. Bei den pH-Werten im Sinne der vorliegenden Erfindung handelt es sich um pH-Werte, die bei einer Temperatur von 22 °C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Es hat sich aber im Rahmen der Untersuchungen zur vorliegenden Erfindung herausgestellt, dass erfindungsgemäß bevorzugte Blondierzubereitungen dadurch gekennzeichnet sind, dass sie zusätzlich ein anorganisches, festes Alkalisierungsmittel enthalten. Dieses Alkalisierungsmittel geht bei Kontakt mit der Feuchtigkeit der Haarsträhne zumindest teilweise in Lösung und stellt so den für Blondiervorgänge erforderlichen alkalischen pH-Wert ein. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Die erfindungsgemäßen Zusammensetzungen enthalten die Alkalisierungsmittel bevorzugt in Mengen von 0,2 bis 25 Gew.-%, insbesondere 0,5 bis 10 Gew.-%.

Eine Ausführungsform der vorliegenden Erfindung ist dadurch gekennzeichnet, dass die kosmetische Blondierzubereitung mindestens 10 Gew.%, vorzugsweise mindestens 25 Gew.% und insbesondere mindestens 50 Gew.-% des Gesamtgewichtes der Folie ausmacht.

Zur Anwendung wird ein oder mehrere der erfindungsgemäßen, mit Blondierzubereitung beaufschlagten Applikator-Folien um ein nasses Bündel keratinischer Fasern, wie zum Beispiel eine nasse Strähne menschlichen Haares, gewickelt. Falls notwendig, kann in einer alternativen Anwendungsform die Blondierzubereitung auf der Folie unmittelbar vor der Anwendung noch zusätzlich mit Wasser befeuchtet werden.

Ein weiterer Gegenstand der vorliegenden Erfindung ist daher ein Verfahren zum Aufhellen von Strähnen keratinischen Fasern, welches dadurch gekennzeichnet ist, dass mindestens ein nasses Bündel der keratinischen Fasern auf eine Applikator-Folie des ersten Erfindungsgegenstands aufgelegt und in die Folie eingewickelt wird. Das Bündel keratinischer Fasern ist dabei insbesondere eine nasse oder feuchte Haarsträhne.

Dabei kann es unerheblich sein, ob die Haarsträhne aus dem nassen Haar separiert wurde und noch hinreichend befeuchtet ist, oder ob die Haarsträhne aus dem trockenen Haar isoliert wurde und unmittelbar vor dem Einwickeln in die Applikator-Folie befeuchtet wurde.

Zur Aufhellung insbesondere längerer Haare ist es jedoch sinnvoll, aus dem noch trockenen Haar ein Bündel von keratinischen Fasern oder eine Strähne zu isolieren, dieses Bündel bzw. Strähne gut zu befeuchten, und dieses so befeuchtete Bündel in die erfindungsgemäßen Applikator-Folien zur Aufhellung einzuwickeln. Zur Befeuchtung der Strähne eignet sich dabei insbesondere eine Sprühflasche.

Eine weitere Ausführungsform des erfindungsgemäßen Verfahrens ist daher dadurch gekennzeichnet, dass als Bündel keratinischer Fasern eine Strähne menschlichen Haars aus dem trockenen Haar isoliert wird, diese Strähne auf der Applikator-Folie mit Wasser eingehend befeuchtet wird und unmittelbar danach in die Folie eingewickelt wird.

Je nach Bedarf und Wunsch des Anwenders können so einzelne oder mehrere verschiedene Strähnen über das gesamte Haar verteilt umwickelt werden. Dadurch können punktuelle, kumulierte oder gleichmäßig verteilte Reflexe und Highlights auf dem Haar erzeugt werden.

Die von der Applikator-Folie umwickelten Strähnen verbleiben je nach gewünschter Aufhellung für eine bestimmte Einwirkzeit bei Raumtemperatur oder bei erhöhter Temperatur zwischen 25 und 45°C. Die Einwirkzeit beträgt zwischen 1 und 60 min, bevorzugt zwischen 5 und 45 min und insbesondere zwischen 10 und 30 min. Anschließend werden die Folien entfernt, die Strähnen mit Wasser oder einer Nachbehandlungszubereitung, wie einem handelsüblichen Konditioniermittel oder Shampoo, gespült und getrocknet.

Eine Ausführungsform des erfindungsgemäßen Verfahrens ist dadurch gekennzeichnet, dass die Folie nach einer Einwirkzeit von 1 bis 45 Minuten von der/den Strähne(n) entfernt und die keratinischen Fasern ausgespült werden.

Ein weiterer Gegenstand der vorliegenden Erfindung ist die Verwendung von mindestens einer Applikator-Folie des ersten Erfindungsgegenstands zur Erzeugung von gezielten, aufgehellten Reflexen im menschlichen Haar. Insbesondere gleichmäßig gefärbtem Haar oder einer natürlich sehr homogenen Haarfarbe können auf diese Weise natürliche, auflockernde Farbreflexe und Highlights erzielt werden. Andererseits sind bei starker Aufhellung so scharfe Kontraste und Akzentuierungen innerhalb der Frisur möglich.

Die Verwendung dieser Applikator-Folien in Kombination oder zeitlich naher Abfolge mit weiteren Farb- und Formveränderungsmitteln, wie beispielsweise oxidativen Aufhell- und/oder Färbemitteln, ist ebenfalls möglich und kann vom Fachmann oder Anwender nach seinen Bedürfnissen und Wünschen dementsprechend eingesetzt werden.

Bezüglich weiterer bevorzugter Ausführungsformen der erfindungsgemäßen Verfahren und Verwendungen gilt mutatis mutandis das zu den erfindungsgemäßen Applikator-Folien Gesagte.

## Patentansprüche

1. Folien-Applikator zur selektiven Aufhellung keratinischer Fasern, umfassend ein blattförmiges Substrat sowie eine darauf aufgebrachte kosmetische Blondierzubereitung, **dadurch gekennzeichnet, dass** die Blondierzubereitung wasserfrei ist und mindestens ein festes Peroxodisulfat-Salz sowie mindestens ein festes Anlagerungsprodukt von Wasserstoffperoxid an organische oder anorganische Verbindungen sowie weiterhin mindestens ein Polymer, ausgewählt aus Cellulose, Celluloseethern, Alginsäure, Xanthan-Gum, Gummi arabicum, Polyvinylalkohol oder Umsetzungsprodukten von Guar-Gum mit Chloressigsäure, mit Ethylenoxid oder mit Propylenoxid, enthält.

2. Folien-Applikator nach Anspruch 1, **dadurch gekennzeichnet, dass** das blattförmige Substrat eine Dicke von 5 µm bis 10 mm, vorzugsweise von 10 µm bis 1 mm, besonders bevorzugt von 15 µm bis 0,5 mm und insbesondere von 20 µm bis 0,1 mm aufweist

3. Folien-Applikator nach einem der Ansprüche 1 oder 2, **dadurch gekennzeichnet, dass** das blattförmige Substrat wenigstens eine Schicht aus Aluminiumfolie umfasst

4. Folien-Applikator nach einem der Ansprüche 1 bis 3, **dadurch gekennzeichnet, dass** das Peroxodisulfat-Salz ausgewählt ist aus Ammoniumperoxodisulfat, Kaliumperoxodisulfat und Natriumperoxodisulfat.

5. Folien-Applikator nach einem der Ansprüche 1 bis 4, **dadurch gekennzeichnet dass** die Blondierzubereitung als Peroxodisulfat-Salz eine Mischung aus mindestens Ammoniumperoxodisulfat und mindestens Kaliumperoxodisulfat und/oder Natriumperoxodisulfat enthält

6. Folien-Applikator nach einem der Ansprüche 1 bis 5, **dadurch gekennzeichnet, dass** das feste Anlagerungsprodukt von Wasserstoffperoxid an organische oder anorganische Verbindungen ausgewählt ist aus Anlagerungsprodukten an Harnstoff, Melamin und Polyvinylpyrrolidinon.

7. Folien-Applikator nach einem der Ansprüche 1 bis 6, **dadurch gekennzeichnet, dass** die kosmetische Blondierzubereitung mindestens 10 Gew.%, vorzugsweise mindestens 25 Gew.% und insbesondere mindestens 50 Gew.-% des Gesamtgewichtes der Folie ausmacht.

8. Verfahren zum Aufhellen von keratinischen Fasern, **dadurch gekennzeichnet, dass** mindestens ein nasses Bündel der keratinischen Fasern auf eine Folie gemäß einem der Ansprüche 1 bis 7 aufgelegt und in die Folie eingewickelt wird, für eine Einwirkzeit in der Applikator-Folie belassen wird, anschließend die Folie entfernt wird und das Bündel der keratinischen Fasern ausgespült wird.

9. Verfahren nach Anspruch 8, **dadurch gekennzeichnet, dass** als Bündel keratinischer Fasern eine Strähne menschlichen Haars aus dem trockenen Haar isoliert wird, diese Strähne auf der Applikator-Folie mit Wasser eingehend befeuchtet wird und unmittelbar danach in die Folie eingewickelt wird.

10. Verfahren nach einem der Ansprüche 8 und 9, **dadurch gekennzeichnet, dass** die Folie nach einer Einwirkzeit von 1 bis 45 Minuten von der/den Strähne(n) entfernt und die keratinischen Fasern ausgespült werden.

11. Verwendung von mindestens einer Applikator-Folie gemäß einem der Ansprüche 1 bis 7 zur Erzeugung von gezielten, aufgehellten Reflexen im menschlichen Haar.

## Claims

1. A foil applicator for selective lightening of keratinic fibers, comprising a sheet-shaped substrate as well as a cosmetic hair-bleaching preparation applied thereonto, **characterized in that** the hair-bleaching preparation is anhydrous and contains at least one solid peroxodisulfate salt as well as at least one solid addition product of hydrogen peroxide with organic or inorganic compounds, as well as furthermore at least one polymer selected from cellulose, cellulose ethers, alginic acid, xanthan gum, gum arabic, polyvinyl alcohol, or reaction products of guar gum with chloroacetic acid, with ethylene oxide, or with propylene oxide.

2. The foil applicator according to Claim 1, **characterized in that** the sheet-shaped substrate has a thickness from 5 µm to 10 mm, preferably from 10 µm to 1 mm, particularly preferably from 15 µm to 0.5 mm, and in particular from 20 µm to 0.1 mm.

3. The foil applicator according to one of Claims 1 or 2, **characterized in that** the sheet-shaped substrate comprises at least one layer made of aluminum foil.

4. The foil applicator according to one of Claims 1 to 3, **characterized in that** the peroxodisulfate salt is selected from ammonium peroxodisulfate, potassium peroxodisulfate, and sodium peroxodisulfate.

5. The foil applicator according to one of Claims 1 to 4, **characterized in that** the hair-bleaching preparation contains, as a peroxodisulfate salt, a mixture of at least ammonium peroxodisulfate and at least potassium peroxodisulfate and/or sodium peroxodisulfate.

6. The foil applicator according to one of Claims 1 to 5, **characterized in that** the solid addition product of hydrogen peroxide with organic or inorganic compounds is selected from addition products with urea, melamine, and polyvinylpyrrolidinone.

7. The foil applicator according to one of Claims 1 to 6, **characterized in that** the cosmetic hair-bleaching preparation accounts for at least 10 wt%, preferably at least 25 wt%, and in particular at least 50 wt% of the total weight of the foil.

8. A method for lightening keratinic fibers, **characterized in that** at least one wet bundle of the keratinic fibers is laid onto a foil in accordance with one of Claims 1 to 7 and wrapped in the foil, left for a contact time in the applicator foil, then the foil is removed and the bundle of keratinic fibers is rinsed out.

9. The method according to Claim 8, **characterized in that** a strand of human hair, as a bundle of keratinic fibers, is isolated from the dry hair, said strand is thoroughly moistened with water on the applicator foil, and immediately thereafter is wrapped in the foil.

10. The method according to one of Claims 8 and 9, **characterized in that** after a contact time from 1 to 45 minutes, the foil is removed from the strand(s) and the keratinic fibers are rinsed out.

11. Use of at least one applicator foil in accordance with one of Claims 1 to 7 to generate selective, lightened highlights in human hair.

## Revendications

1. Applicateur en film pour la décoloration sélective de fibres kératiniques, comprenant un substrat en forme de feuille ainsi qu'une préparation cosmétique de décoloration appliquée par-dessus, **caractérisé en ce que** la préparation de décoloration est anhydre et contient au moins un sel solide de peroxodisulfate ainsi qu'au moins un produit solide de fixation par addition de peroxyde d'hydrogène à des composés organiques ou inorganiques, et en outre au moins un polymère choisi parmi la cellulose, des éthers cellulosiques, l'acide alginique, la gomme de xanthane, la gomme arabique, l'alcool polyvinylique ou des produits réactionnels de la gomme de guar avec de l'acide chloroacétique, avec de l'oxyde d'éthylène ou avec de l'oxyde de propylène.

2. Applicateur en film selon la revendication 1, **caractérisé en ce que** le substrat en forme de feuille présente une épaisseur de 5 µm à 10 mm, de préférence de 10 µm à 1 mm, de manière particulièrement préférée de 15 µm à 0,5 mm et en particulier de 20 µm à 0,1 mm.

3. Applicateur en film selon la revendication 1 ou 2, **caractérisé en ce que** le substrat en forme de feuille comprend au moins une couche constituée d'un film d'aluminium.

4. Applicateur en film selon l'une quelconque des revendications 1 à 3, **caractérisé en ce que** le sel de peroxodisulfate est choisi parmi le peroxodisulfate d'ammonium, le peroxodisulfate de potassium et le peroxodisulfate de sodium.

5. Applicateur en film selon l'une quelconque des revendications 1 à 4, **caractérisé en ce que** la préparation de décoloration contient, à titre de sel de peroxodisulfate, un mélange d'au moins un peroxodisulfate d'ammonium et d'au moins un peroxodisulfate de potassium et/ou de peroxodisulfate de sodium.

6. Applicateur en film selon l'une quelconque des revendications 1 à 5, **caractérisé en ce que** le produit solide de fixation par addition de peroxyde d'hydrogène à des composés organiques ou inorganiques est choisi parmi des produits de fixation par addition à de l'urée, à de la mélamine et à de la polyvinylpyrrolidinone.

7. Applicateur en film selon l'une quelconque des revendications 1 à 6, **caractérisé en ce que** la préparation cosmétique de décoloration représente au moins 10 % en poids, de préférence au moins 25 % en poids et en particulier au moins 50 % en poids du poids total du film.

8. Procédé pour la décoloration de fibres kératiniques, **caractérisé en ce qu'**on dépose au moins un faisceau humide des fibres kératiniques sur un film selon au moins une des revendications 1 à 7 et on l'enroule dans le film, on laisse agir pendant un temps de réaction dans le film faisant office d'applicateur, on retire ensuite le film et on rince le faisceau de fibres kératiniques.

9. Procédé selon la revendication 8, **caractérisé en ce que**, à titre de faisceau de fibres kératiniques, on isole une mèche de cheveux humains à partir des cheveux séchés, on humidifie avec insistance cette mèche avec de l'eau sur le film faisant office d'applicateur et on l'enroule directement après dans le film.

10. Procédé selon l'une quelconque des revendications 8 et 9, **caractérisé en ce qu'**on retire le film de la mèche/des mèches après un temps de réaction de 1 à 45 minutes et on rince les fibres kératiniques.

11. Utilisation d'au moins un film faisant office d'applicateur selon l'une quelconque des revendications 1 à 7 pour l'obtention de reflets décolorés ciblés dans les cheveux humains.
